# EUROPÄISCHE PATENTSCHRIFT

(11) **EP 0 889 023 B1**
(45) Veröffentlichungstag und Bekanntmachung des Hinweises auf die Patenterteilung: **11.10.2000**
(21) Anmeldenummer: 98111419.2
(22) Anmeldetag: 22.06.1998
(51) Int. Cl.: C07C 67/03, C07C 69/33, C07C 69/52

(54) **Verfahren zur Herstellung von Mischungen aus Sorbitmonoestern, Sorbitdiestern und Partialglyceriden**
Process for the preparation of mixtures of sorbitolmonoesters, sorbitoldiesters and partial glycerides
Procédé de préparation de mélanges de monoesters de sorbitol, de diesters de sorbitol et de glycérides partiels

(30) Priorität: 01.07.1997 DE 19727950
(43) Veröffentlichungstag der Anmeldung: 07.01.1999
(73) Patentinhaber: Clariant GmbH, 65929 Frankfurt am Main (DE)
(72) Erfinder: Prossel, Günter, Dr., 84508 Burgkirchen (DE); Papenfuhs, Bernd, Dr., 63179 Obertshausen (DE)

(56) Entgegenhaltungen:
- DE-A- 2 313 543
- GB-A- 966 541

## Beschreibung

Die Erfindung betrifft ein ökonomisches Verfahren zur verbesserten Herstellung von Mischungen aus Sorbitmonoestern, Sorbitdiestern und Partialglyceriden. Im Kosmetikbereich und auch in Nahrungsmitteln werden häufig Fettsäuremonoglyceride und Fettsäurediglyceride als Emulgatoren verwendet. Partialglyceride erhält man üblicherweise durch Umesterung von Triglyceriden mit Glycerin.

In der DE-A-23 13 543 wird ein Verfahren zur Umsetzung von Triglyceriden mit Sorbit beschrieben, das jedoch in bezug auf die Reinheit der Produkte nicht befriedigt.

Aus EP-B-108 999 ist ein Verfahren zur Herstellung von Carbonsäureestern von Hexiten bekannt, wobei eine Mischung aus Hexit und Carbonsäure in Gegenwart eines alkalischen Katalysators unter Durchleiten von Inertgas bei einer Temperatur von 210 bis 250 °C umgesetzt wird und das entstehende Wasser mit Hilfe eines Abscheiders entfernt wird. Nachteil dieses Verfahrens ist, daß unter den drastischen Reaktionsbedingungen eine Wasserabspaltung unter Bildung von Anhydrohexoseestern erfolgt, die in hohen Anteilen cyclisiert sind. Demzufolge weisen die so erhaltenen Carbonsäurehexoseester durchwegs zu niedrige OH-Zahlen auf.

Ein wesentliches Ziel der vorliegenden Erfindung war die Ermittlung von schonenden Reaktionsbedingungen, unter denen fast ausschließlich nichtcyclische Sorbitester entstehen, daß heißt, daß die Bildung von Anhydrosorbitestern vermieden wird. Bei der Umsetzung eines Triglycerids mit Sorbit handelt es sich um eine alkalisch katalysierte partielle Alkoholyse des Triglycerids unter gleichzeitiger Veresterung des Sorbits.

Als alkalische Katalysatoren können Alkalimetallhydroxide und -carbonate sowie Alkalimetallalkoholate oder auch hochsiedende tertiäre Amine verwendet werden. Als Triglyceride werden Kokosfett, Palmöl, Palmkernöl, Rapsöl, Sojabohnenöl und Sonnenblumenöl jeweils in raffinierter Qualität und - bevorzugt - in hydrierter Qualität verwendet. Die meisten Versuche wurden mit raffiniertem und/oder hydriertem Kokosfett durchgeführt. Zur besseren Handhabbarkeit können Katalysatoren wie Alkalialkoholate in alkoholischer Lösung oder Kaliumcarbonat in wäßriger Lösung zur Mischung der Edukte gegeben werden. Diese Lösemittel können vor der eigentlichen Reaktion durch Aufheizen auf 80 bis 100 °C im Vakuum vollständig entfernt werden. Die Reaktion wird unter Stickstoff als Schutzgas durchgeführt.

Typischerweise ist bei einem Molverhältnis von Sorbit zu Triglycerid von 1 : 1 und einer Katalysatormenge von 0,5 bis 2,0 Gew.-%, bezogen auf die Mischung der Edukte, eine Reaktionszeit von 10 bis 20 Stunden bei 140 bis 150 °C zur vollständigen Umsetzung erforderlich. Überraschend wurde nun gefunden, daß man durch Zugabe eines wie vorstehend beschrieben erhaltenen Reaktionsprodukts in geringer Menge zu der Mischung aus Sorbit, Triglycerid und Katalysator sowohl die Reaktionszeit verkürzen als auch die Reaktionstemperatur senken kann. Die zugesetzte wirksame Menge an Reaktionsprodukt beträgt 1 bis 10 Gew.-%, vorzugsweise 3 bis 7 Gew.-%, jeweils bezogen auf die eingesetzte Mischung. Die zugesetze Menge an Reaktionsprodukt wird im folgenden als "Eigenemulgator" bezeichnet. Durch die erfindungsgemäße Zugabe von Eigenemulgator läßt sich die erforderliche Reaktionstemperatur im Mittel um circa 20 °C senken, wobei gleichzeitig im Mittel die Reaktionszeit halbiert werden kann. Die niedrigere Reaktionstemperatur führt zu weniger Nebenprodukten, also reineren Produkten. Der größte Vorteil ist jedoch die Einsparung an Arbeitszeit, weil in der selben Herstellanlage nun doppelt soviel Produkt erhalten wird. De facto wird also die Raum-Zeit-Ausbeute verdoppelt. Unter Verwendung von Kaliumcarbonat als basischem Katalysator werden ohne Eigenemulgator allgemein längere Reaktionszeiten und/oder höhere Reaktionszeiten als mit Natriummethanolat benötigt. Mit Eigenemulgator entfällt dieser Nachteil. Dies ist insofern vorteilhaft, als mit Kaliumcarbonat meist hellere Produkte erhalten werden.

Es wurde weiterhin gefunden, daß Verbindungen der Formel (I) 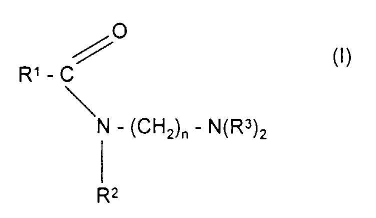 worin bedeuten:
- R¹: eine Alkyl- oder Alkenylgruppe mit 6 bis 13 C-Atomen,
- R²: eine Alkylgruppe mit 1 bis 6 C-Atomen oder ein H-Atom,
- R³: eine Alkylgruppe mit 1 bis 6 C-Atomen,
- n: eine Zahl von 1 bis 6,
die Reaktion von Sorbit mit Triglyceriden effizient in homogener Phase katalysieren. Die Menge an alkalischem Katalysator kann dadurch drastisch reduziert werden, beziehungsweise es ist überhaupt kein alkalischer Katalysator mehr erforderlich. Die Menge an Verbindung der Formel (I) beträgt ebenfalls 1 bis 10, vorzugsweise 3 bis 7 Gew.-%, bezogen auf die eingesetzte Mischung.

Bei dem genannten erfindungsgemäßen Verfahren kann auch von einem 70%igem Sorbitsirup ausgegangen werden. In diesem Fall wird das enthaltene Wasser durch kurzzeitiges Erhitzen auf 120 °C bei reduziertem Druck (circa 30 mbar) entfernt. Nach Abkühlen der Sorbitschmelze auf 80 °C wird zunächst der alkalische Katalysator zugegeben, dann der Eigenemulgator und schließlich das flüssige Triglycerid. Die eigentliche Reaktion wird dann bei 115 bis 135°C, vorzugsweise 120 bis 130°C durchgeführt. Die typischen Produkte sind bei Raumtemperatur pastenförmig bis wachsartig und beige bis hellbraun gefärbt. Der Restgehalt an Sorbit liegt in der Regel bei 4 bis 5 %, der Gehalt an Anhydrosorbit bei 0,1 bis 1,0 %. Die erfindungsgemäß hergestellten Produkte sind besonders hautmild und eignen sich daher insbesondere als Emulgatoren für kosmetische Formulierungen.

Im folgenden wird das neue Verfahren an Beispielen erläutert.

Hierbei entsprechen die Beispiele 1a, 2a, 3a, 4a und 5a dem bisher besten Stand der Technik. Die Beispiele 1b, 2b, 3b, 4b und 5b beschreiben das neue verbesserte Verfahren unter Zugabe von 5 Gew.-% Eigenemulgator. In Beispiel 3c wird ein Amidamin der Formel (I) verwendet, das ebenfalls mit 5 Gew.-% eingesetzt wird.

### Vergleichsbeispiel 1a

### (Umsetzung von Sorbit mit raffiniertem Kokosfett)

In einem 2-l-Vierhalskolben mit Rührer, Kühler, Stickstoffzufluß und Thermometer werden 660 g (1 mol) raffiniertes Kokosfett vorgelegt und auf 120 °C erwärmt. 182 g (1 mol) Sorbit und 8,4 g NaOCH₃ (1 Gew.-%, bezogen auf Gesamteinwaage) werden zugegeben und auf 140 °C erwärmt. Nach der gaschromatographischen Analyse (GC) ist die Reaktion nach 20 Stunden bei 140 °C unter N₂ beendet. Der Rest-Sorbitgehalt beträgt 5,2 Gew.-%. Als Umsetzungsprodukt entsteht in quantitativer Ausbeute 850 g einer Mischung aus Sorbitmonoester, Sorbitdiester, Fettsäuremonoglycerid und Fettsäurediglycerid als Hauptkomponenten mit einem Schmelzbereich von 39 bis 48 °C.

| | |
|---|---|
| lodfarbzahl | 5 |
| OH-Zahl | 359 mg KOH/g |
| Verseifungszahl | 198 mg KOH/g |

### Beispiel 1b

### (Versuch mit Zugabe von 5 % Eigenemulgator)

In entsprechender Weise, wie in Beispiel 1a mit einer Zugabe von 43 g Eigenemulgator (5 Gew.%, bezogen auf Gesamteinwaage) zum Reaktionsgemisch. Nach der GC-Analyse ist die Reaktion nach 7 Stunden bei 120 °C unter N₂ beendet. Der Rest-Sorbitgehalt beträgt 5,0 Gew.-%. Als Umsetzungsprodukt entsteht in quantitativer Ausbeute 893 g einer Sorbitester/Partialglycerid-Mischung mit einem Schmelzbereich von 39 bis 47 °C.

| | |
|---|---|
| lodfarbzahl | 4 |
| OH-Zahl | 355 mg KOH/g |
| Verseifungszahl | 197 mg KOH/g |

### Vergleichsbeispiel 2a

### (Umsetzung von Sorbit mit hydriertem Kokosfett)

In entsprechender Weise, wie in Beispiel 1a beschrieben, wird bei diesem Beispiel 655 g (1 mol) hydriertes Kokosfett, 182 g (1 mol) Sorbit und 8,4 g Natriummethylat (1 Gew.-%, bezogen auf Gesamteinwaage) eingesetzt. Nach der GC-Analyse ist die Reaktion nach 11 Stunden bei 140 °C unter N₂ beendet. Der Rest-Sorbitgehalt beträgt 5,3 Gew.-%. Als Umsetzungsprodukt entsteht in quantitativer Ausbeute 845 g einer Sorbitester/Partialglycerid-Mischung mit einem Schmelzbereich von 35 bis 42 °C.

| | |
|---|---|
| lodfarbzahl | 4 |
| OH-Zahl | 380 mg KOH/g |
| Verseifungszahl | 197 mg KOH/g |

### Beispiel 2b

### (Versuch mit Zugabe von 5 % Eigenemulgator)

In entsprechender Weise, wie Beispiel 2a mit einer Zugabe von 42 g Eigenemulgator (5 Gew.-%, bezogen auf Gesamteinwaage) zum Reaktionsgemisch. Nach der GC-Analyse ist die Reaktion nach 7 Stunden bei 120 °C unter N₂ beendet. Der Rest-Sorbitgehalt beträgt 5,2 Gew.-%. Als Umsetzungsprodukt entsteht in quantitativer Ausbeute 887 g einer Sorbitester/Partialglycerid-Mischung mit einem Schmelzbereich von 35 bis 42°C.

| | |
|---|---|
| lodfarbzahl | 3 |
| OH-Zahl | 383 mg KOH/g |
| Verseifungszahl | 197 mg KOH/g |

### Vergleichsbeispiel 3a

### (Umsetzung von Sorbit mit hydriertem Kokosfett, Katalysator: K₂CO₃)

In entsprechender Weise, wie in Beispiel 1a beschrieben, wird bei diesem Beispiel 655 g (1 mol) hydriertes Kokosfett, 182 g (1 mol) Sorbit und 8,4 g Kaliumcarbonat (1 Gew.-%, bezogen auf Gesamteinwaage) eingesetzt. Nach der GC-Analyse ist die Reaktion nach 13 Stunden bei 150 °C unter N₂ beendet. Der Rest-Sorbitgehalt beträgt 4,2 Gew.-%. Als Umsetzungsprodukt entsteht in quantitativer Ausbeute 845 g einer Sorbitester/Partialglycerid-Mischung mit einem Schmelzbereich von 38 bis 46°C.

| | |
|---|---|
| lodfarbzahl | 4 |
| OH-Zahl | 364 mg KOH/g |
| Verseifungszahl | 197 mg KOH/g |

### Beispiel 3b

### (Versuch mit Zugabe von 5 % Eigenemulgator)

In entsprechender Weise, wie Beispiel 3a mit einer Zugabe von 42 g Eigenemulgator (5 Gew.-%, bezogen auf Gesamteinwaage) zum Reaktionsgemisch. Nach der GC-Analyse ist die Reaktion nach 9,5 Stunden bei 130 °C unter N₂ beendet. Der Rest-Sorbitgehalt beträgt 4,5 Gew.-%. Als Umsetzungsprodukt entsteht in quantitativer Ausbeute 887 g einer Sorbitester/Partialglycerid-Mischung mit einem Schmelzbereich von 38 bis 45°C.

| | |
|---|---|
| lodfarbzahl | 4 |
| OH-Zahl | 361 mg KOH/g |
| Verseifungszahl | 198 mg KOH/g |

### Beispiel 3c

### (Versuch mit Zugabe von 5 % Kokosfettsäureamidopropyl-N,N-dimethylamin)

In entsprechender Weise, wie Beispiel 3a mit einer Zugabe von 42 g Kokosfettsäureamidopropyl-N,N-dimethylamin (5 Gew.-%, bezogen auf Gesamteinwaage) zum Reaktionsgemisch. Nach der GC-Analyse ist die Reaktion nach 11 Stunden bei 130 °C unter N₂ beendet. Der Rest-Sorbitgehalt beträgt 3,8 Gew.-%. Als Umsetzungsprodukt entsteht in quantitativer Ausbeute 887 g einer Sorbitester/Partialglycerid-Mischung mit einem Schmelzbereich von 38 bis 45°C.

| | |
|---|---|
| lodfarbzahl | 4 |
| OH-Zahl | 379 mg KOH/g |
| Verseifungszahl | 200 mg KOH/g |

### Vergleichsbeispiel 4a

### (Umsetzung von Sorbit mit Palmkernöl)

In entsprechender Weise, wie in Beispiel 1a beschrieben, wird bei diesem Beispiel 665 g (1 mol) Palmkernöl, 182 g (1 mol) Sorbit und 8,5 g Natriummethylat (1 Gew.-%, bezogen auf Gesamteinwaage) eingesetzt. Nach der GC-Analyse ist die Reaktion nach 12 Stunden bei 130 °C unter N₂ beendet. Der Rest-Sorbitgehalt beträgt 4,2 Gew.-%. Als Umsetzungsprodukt entsteht in quantitativer Ausbeute 856 g einer Sorbitester/Partialglycerid-Mischung mit einem Schmelzbereich von 40 bis 48°C.

| | |
|---|---|
| lodfarbzahl | 3 |
| OH-Zahl | 382 mg KOH/g |
| Verseifungszahl | 190 mg KOH/g |

### Beispiel 4b

### (Versuch mit Zugabe von 5 % Eigenemulgator)

In entsprechender Weise, wie Beispiel 4a mit einer Zugabe von 43 g Eigenemulgator (5 Gew.-%, bezogen auf Gesamteinwaage) zum Reaktionsgemisch. Nach der GC-Analyse ist die Reaktion nach 7 Stunden bei 120 °C unter N₂ beendet. Der Rest-Sorbitgehalt beträgt 4,7 Gew.-%. Als Umsetzungsprodukt entsteht in quantitativer Ausbeute 899 g einer Sorbitester/Partialglycerid-Mischung mit einem Schmelzbereich von 40 bis 48°C.

| | |
|---|---|
| lodfarbzah | 3 |
| OH-Zahl | 378 mg KOH/g |
| Verseifungszahl | 189 mg KOH/g |

### Vergleichsbeispiel 5a

### (Umsetzung von Sorbit mit Sojabohnenöl)

In entsprechender Weise, wie in Beispiel 1a beschrieben, wird bei diesem Beispiel 876 g (1 mol) Sojabohnenöl, 182 g (1 mol) Sorbit und 10,6 g Kaliumcarbonat (1 Gew.-%, bezogen auf Gesamteinwaage) eingesetzt. Nach der GC-Analyse ist die Reaktion nach 13 Stunden bei 140 °C unter N₂ beendet. Der Rest-Sorbitgehalt beträgt 4,0 Gew.-%. Als Umsetzungsprodukt entsteht in quantitativer Ausbeute 1069 g einer Sorbitester/Partialglycerid-Mischung mit einem Schmelzbereich von 17 bis 23°C.

| | |
|---|---|
| lodfarbzahl | 5 |
| OH-Zahl | 283 mg KOH/g |
| Verseifungszahl | 155 mg KOH/g |

### Beispiel 5b

### (Versuch mit Zugabe von 5 % Eigenemulgator)

In entsprechender Weise, wie Beispiel 5a mit einer Zugabe von 53 g Eigenemulgator (5 Gew.-%, bezogen auf Gesamteinwaage) zum Reaktionsgemisch. Nach der GC-Analyse ist die Reaktion nach 7 Stunden bei 120°C unter N₂ beendet. Der Rest-Sorbitgehalt beträgt 4,3 Gew.-%. Als Umsetzungsprodukt entsteht in quantitativer Ausbeute 1122 g einer Sorbitester/Partialglycerid-Mischung mit einem Schmelzbereich von 17 bis 23 °C.

| | |
|---|---|
| lodfarbzahl | 4 |
| OH-Zahl | 281 mg KOH/g |
| Verseifungszahl | 156 mg KOH/g |

## Patentansprüche

1. Verfahren zur direkten Herstellung von Mischungen, die größtenteils Sorbitmonoester, Sorbitdiester und Partialglyceride enthalten, unter Vermeidung der Bildung cyclischer Sorbitester, durch Umsetzung von Sorbit mit Triglyceriden in Gegenwart mindestens eines alkalischen Katalysators, dadurch gekennzeichnet, daß als Cokatalysator für die genannte Umsetzung verwendet wird:
a) eine Teilmenge des Produkts der obigen Umsetzung als Eigenemulgator und/oder
b) ein Amidamin der nachstehenden Formel (I) 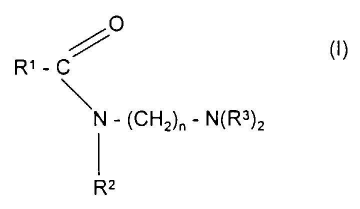 worin bedeuten:
R¹ eine Alkyl- oder Alkenylgruppe mit 6 bis 13 C-Atomen,
R² eine Alkylgruppe mit 1 bis 6 C-Atomen oder ein H-Atom,
R³ eine Alkylgruppe mit 1 bis 6 C-Atomen,
n eine Zahl von 1 bis 6.

2. Verfahren zur Herstellung von Mischungen nach Anspruch 1, dadurch gekennzeichnet, daß die zugesetzte Menge an Eigenemulgator und/oder Amidamin der Formel (I) 1 bis 10 Gew.-%, vorzugsweise 3 bis 7 Gew.-%, beträgt.

3. Verfahren gemäß den Ansprüchen 1 und 2, dadurch gekennzeichnet, daß die Reaktion bei 115 bis 135 °C, vorzugsweise bei 120 bis 130 °C, unter Stickstoffatmosphäre durchgeführt wird.

## Claims

1. A process for the direct preparation of mixtures which comprise mostly sorbitol monoesters, sorbitol diesters and partial glycerides, avoiding the formation of cyclic sorbitol esters, by reaction of sorbitol with triglycerides in the presence of at least one alkaline catalyst, wherein the cocatalyst used for said reaction is:
a) a part amount of the product of the above reaction as a self-emulsifier
and/or
b) an amidoamine of the following formula (I) 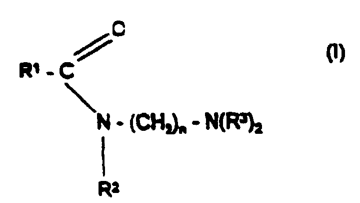 in which:
R¹ is an alkyl or alkenyl group having 6 to 13 carbon atoms,
R² is an alkyl group having 1 to 6 carbon atoms or an H atom,
R³ is an alkyl group having 1 to 6 carbon atoms and
n is a number from 1 to 6.

2. The process for the preparation of mixtures as claimed in claim 1, wherein the amount of self-emulsifier and/or amidoamine of the formula (I) added is 1 to 10% by weight, preferably 3 to 7% by weight.

3. The process as claimed in claims 1 and 2, wherein the reaction is carried out at 115 to 135°C, preferably at 120 to 130°C, under a nitrogen atmosphere.

## Revendications

1. Procédé pour la préparation directe de mélanges, qui contiennent pour la plus grande part des monoesters de sorbitol, des diesters de sorbitol et des glycérides partiels, en évitant la formation d'esters de sorbitol cycliques, par réaction de sorbitol avec des triglycérides en présence d'au moins un catalyseur alcalin, caractérisé en ce que, on utilise comme co-catalyseur pour la réaction citée :
a) une quantité partielle du produit de la réaction ci-dessus mentionnée comme agent autoémulsionnant et/ou
b) une amidoamine de formule (I) suivante 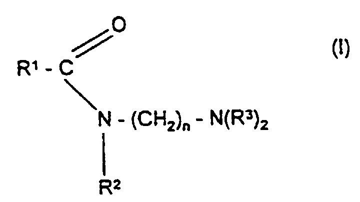
dans laquelle :
R¹ représente un groupe alkyle ou alcényle ayant de 6 à 13 atomes de carbone,
R² représente un groupe alkyle ayant de 1 à 6 atomes de carbone ou un atome d'hydrogène,
R³ représente un groupe alkyle ayant de 1 à 6 atomes de carbone,
n représente un nombre variant de 1 à 6.

2. Procédé pour la préparation de mélanges selon la revendication 1, caractérisé en ce que, la quantité additionnelle d'agent autoémulsionnant et/ou d'amidoamine de formule (I) s'élève de 1 à 10% en poids, de préférence de 3 à 7% en poids.

3. Procédé selon les revendications 1 et 2, caractérisé en ce que, la réaction est réalisée entre 115 et 135°C, de préférence entre 120 et 130°C, sous atmosphère d'azote.
